# EUROPEAN PATENT SPECIFICATION

(11) **EP 0 651 794 B1**
(45) Date of publication and mention of the grant of the patent: **30.09.2009**
(21) Application number: 93914652.8
(22) Date of filing: 06.07.1993
(51) Int. Cl.: C12N 9/28, C12N 9/30, C11D 3/386

(54) **MUTANT $g(a)-AMYLASE, DETERGENT AND DISH WASHING AGENT**
MUTIERTE -g(a)-AMYLASE, WASCHMITTEL UND GESCHIRRSPÜLMITTEL
ALPHA-AMYLASE MUTANTE, DETERGENT ET AGENT DE LAVAGE DE VAISSELLE

(30) Priority: 23.07.1992 DK 94692; 16.12.1992 DK 150392; 15.03.1993 DK 29293
(43) Date of publication of application: 10.05.1995
(60) Divisional application: 06125226.8
(73) Proprietor: Novozymes A/S, 2880 Bagsvaerd (DK)
(72) Inventor: SVENDSEN, Allan, DK-3460 Birkeroed (DK); BISGARD-FRANTZEN, Henrik, DK-2800 Lyngby (DK)
(74) Representative: Rasmussen, Preben
(86) International application number: PCT/DK1993/000230
(87) International publication number: WO 1994/002597

(56) References cited:
- EP-A- 0 130 756
- WO-A-89/07642
- WO-A-91/16423
- The Journal of Biological Chemistry, Volume 260, No. 11, June 1985, DAVID A. ESTELL et al., "Engineering an Enzyme by Site directed Mutagenesis to be Resistant to Chemical Oxidation", page 6518 - page 6521, see 6518, left column, lines 1-6, fig. 2, XP000576091

## Description

The invention comprises a mutant α-amylase, a detergent and a dish washing agent.

Mutant amylases with improved oxidation stability, wherein one or more methionines have been mutated into cysteins or chemically modified cysteins are described in WO 91/16423. The reason for research in this field is the need for oxidation stable α-amylases as additives for detergents and dish washing agents, due to the presence of strong oxidizing agents in the detergents and the dish washing agents, as explained in more detail in WO 91/16423.

It has been found that the activity level and the stability in the presence of oxidizing agents of the prior art mutant amylases is open to improvement, and thus, the purpose of the invention is the provision of a mutant α-amylase with an improved activity level and an improved stability in the presence of oxidizing agents in comparison to the prior art mutant amylases. In this context the term "stability in the presence of oxidizing agents" refers both to the storage stability of the α-amylase during storage of the α-amylase product and during storage of the α-amylase containing detergent or the α-amylase containing dish washing agent, and to the stability in the washing solution or dish washing solution during the washing process or dish washing process, and furthermore to the stability of the α-amylase during hydrolysis of starch in the presence of hydrogen peroxide or other bleaching agents, e.g. during desizing in the textile industry.

According to the invention, there is provided a mutant alpha-amylase as defined in claim 1, a detergent as defined in claim 3 and a dish washing agent as defined in claim 4.

Surprisingly it has been found that the mutant α-amylase according to the invention exhibits a better activity level and a better stability in the presence of oxidizing agents than the prior art mutant amylases.

The mutant α-amylase according to the invention is *B. licheniformis* α-amylase, *B*. *amyloliquefaciens* α-amylase or *B*. *stearothermophilus* α-amylase. These α-amylases are all well characterized and their entire amino acid sequence is described. It is to be understood that an α-amylase, which is identical to the *B. licheniformis* α-amylase identified in FR 2665178, except for the fact that it exhibits an arginin residue in position 23 instead of the lysin residue, also belongs to the α-amylases, which are basis for the mutant α-amylases according to the invention.

A preferred embodiment of the claimed mutant α-amylase according to the invention is characterized by the fact that the methionine amino acid residues is exchanged with a Leu. In this embodiment a very satisfactory activity level and stability in the presence of oxidizing agents is obtained.

Also, the invention comprises a detergent, which is characterized by the fact that it comprises the mutant α-amylase according to the invention. Thus, according to the the invention, the mutant α-amylase may be added as a component of a detergent composition. As such, it may be included in the detergent composition in the form of a detergent additive. The detergent composition as well as the detergent additive may additionally comprise one or more other enzymes conventionally used in detergents, such as proteases, lipases, cellulases, oxidases or peroxidases.

In a specific aspect, the invention provides a detergent additive. The enzymes may be included in a detergent composition by adding separate additives containing one or more enzymes, or by adding a combined additive comprising all of these enzymes.

Preferably, the detergent additive, i.e. a separated additive or a combined additive, is provided in the form of a granulate, preferably a non-dusting granulate, a liquid, in particular a stabilized liquid, a slurry, or in a protected form.

Dust free granulates may be produced, e.g. as disclosed in US 4,106,991 and US 4,661,452 (both to Novo Industri A/S) and may optionally bet coated by methods known in the art. The detergent enzymes may be mixed before or after granulation.

Liquid enzyme preparations may, for instance, be stabilized by adding a polyol such as e.g. propylene glycol, a sugar or sugar alcohol, lactic acid or boric acid, according to established methods. Other enzyme stabilizers are well known in the art.

Protected enzymes may be prepared according to the method disclosed in EP 238,216 A.

The detergent composition of the invention may be in any convenient form, e.g. as powder, granules or liquid. A liquid detergent may be aqueous, typically containing up to 90% water and 0-20% organic solvent.

The detergent composition comprises a surfactant which may be anionic, non-ionic, cationic, amphoteric or a mixture of these types. The detergent will usually contain 0-50% anionic surfactant such as linear alkyl benzene sulphonate (LAS), alpha-olefin sulphonate (AOS), alkyl sulphate (AS), alcohol ethoxy sulphate (AES) or soap. It may also contain 0-40% non-ionic surfactant such as nonyl phenol ethoxylate or alcohol ethoxylate. Furthermore, it may contain a polyhydroxy fatty acid amide surfactant (e.g. as described in WO 92/06154).

The pH (measured in aqueous detergent solution) will usually be neutral or alkaline, e.g. 7-11. The detergent may contain 1-40% of a detergent builder such as zeolite, phosphate, phosphonate, citrate, NTA, EDTA or DTPA, alkenyl succinic anhydride, or silicate, or it may be unbuilt (i.e. essentially free of a detergent builder). It may also contain other conventional detergent ingredients, e.g. fabric conditioners, foam boosters, bleaching agents, e.g. perborate, percarbonate, tetraacetyl ethylene diamine (TAED), or nonanoyloxybenzene sulfonate (NOBS), anti-corrosion agents, soil-suspending agents, sequestering agents, anti-soil redeposition agents, stabilizing agents for the enzyme(s), foam depressors, dyes, bactericides, optical brighteners or perfumes.

Particular forms of detergent compositions within the scope of the invention include:
a) A detergent composition formulated as a detergent powder containing phosphate builder, anionic surfactant, nonionic surfactant, silicate, alkali to adjust to desired pH in use, and neutral inorganic salt.
b) A detergent composition formulated as a detergent powder containing zeolite builder, anionic surfactant, nonionic surfactant, acrylic or equivalent polymer, silicate, alkali to adjust to desired pH in use, and neutral inorganic salt.
c) A detergent composition formulated as an aqueous detergent liquid comprising anionic surfactant, nonionic surfactant, humectant, organic acid, alkali, with a pH in use adjusted to a value between 7 and 10.5.
d) A detergent composition formulated as a nonaqueous detergent liquid comprising a liquid nonionic surfactant consisting essentially of linear alkoxylated primary alcohol, phosphate builder, alkali, with a pH in use adjusted to a value between about 7 and 10.5.
e) A detergent composition formulated as a detergent powder in the form of a granulate having a bulk density of at least 600 g/l, containing anionic surfactant and nonionic surfactant, low or substantially zero neutral inorganic salt, phosphate builder, and sodium silicate.
f) A detergent composition formulated as a detergent powder in the form of a granulate having a bulk density of at least 600 g/l, containing anionic surfactant and nonionic surfactant, low or substantially zero neutral inorganic salt, zeolite builder, and sodium silicate.
g) A detergent composition formulated as a detergent powder containing anionic surfactant, nonionic surfactant, acrylic polymer, fatty acid soap, sodium carbonate, sodium sulphate, clay particles, and sodium silicate.
h) A liquid compact detergent comprising 5-65% by weight of surfactant, 0-50% by weight of builder and 0-30% by weight of electrolyte.

It is at present contemplated that, in the detergent composition of the invention, the mutant α-amylase may be added in an amount corresponding to 0.001-100 mg of enzyme per liter of wash liquor.

Also, the invention comprises a dish washing agent, which is characterized by the fact that it comprises the mutant α-amylase according to the invention. All dish washing agent formulations can be used in combination with the mutant α-amylase according to the invention.

Both the detergent according to the invention and the dish washing agent according to the invention may comprise oxidizing agents, which may be an activator, a bleaching agent or an oxidizing enzyme. The bleaching agent may be a chlorine containing agent, preferably a hypochlorit generating agent, a percarbonate, or a perborate.

From the Journal of Biological Chemistry; Vol. 260, No. 11, pages 6518-6521 it appears that site-directed mutagenesis can be employed to alter critical residues in proteins which are susceptible to chemical oxidation, and that methionine 222 is a primary site for oxidative inactivation of subtilisin. This citation, however, does not describe mutants of α-amylase, whereas the invention is strictly limited to mutants of α-amylase.

The amino acid sequence for the *B*. *licheniformis* α-amylase appears from J. Bacteriol. 166, 635-643, 1986, FR 2665178 or EP 410498. Thus, the methionine numbers are: 8, 15, 197, 256, 304, 366, and 438.

The amino acid sequence for the *B. amyloliquefaciens* α-amylase appears from J. Biol. Chem. 258, 1007-1013, 1983. Thus, the methionine numbers are: 6, 197, 256, 304, 366, and 438.

The amino acid sequence for the *B. stearothermophilus* α-amylase appears from J. Bacteriol. 166, 635-643, 1986. Thus, the methionine numbers are: 8, 9, 97, 200, 206, 284, 307, 311, 316, and 437.

Once an exchange pattern is decided upon the genetically engineered protein can be synthesized without any inventive effort according to prior art methods.

FR 2665178 describes a thermostable variant of the α-amylase from *B. licheniformis*. This variant, however, does not involve a methionine exchange, but an exchange in the neighbourhood of histidine 133, and also, the oxidative stability of this prior art α-amylase is inferior in comparison to the oxidative stability of the α-amylase mutant according to the invention.

Documentation for the surprising properties of the mutant α-amylase according to the invention, e.g. the improved activity level and the improved stability in the presence of oxidizing agents, will be presented in the following.

### Documentation for improved stability in the presence of oxidizing agents

Raw filtered culture broths with different Termamyl^{⊗} mutants indicated below are diluted to an amylase activity of 100 NU/ml (the NU amylase activity unit is defined in AF 207/1, which is available on request from Novo Nordisk A/S, Novo Allé, DK-2880 Bagsvaerd, Denmark) in 50 mM of a Britton-Robinson buffer at pH 9.0 and incubated at 40°C. Subsequently H₂O₂ is added to a concentration of 200 mM, and the pH value is re-adjusted to 9.0. The activity is now measured after 15 seconds and after 5, 15, and 30 minutes. The results appear from the following table 1, in which the amylase mutant is identified by means of the one letter amino acid system. Thus, M197A means the Termamyl^{®} mutant, in which the methionine in position 197 is exchanged with alanine. It clearly appears from Table 1 that the prior art Cystein mutant (M197C) exhibits a very low stability, even lower than the stability of Termamyl^{®}.

The values in the following Table 1 is the OD₆₂₀ absorption values, which are taken as an indication of the activity.

**Table 1**

| OD₆₂₀ absorption values | | | | |
|---|---|---|---|---|
| | 15 seconds (0) | 5 minutes | 15 minutes | 30 minutes |
| M197A | 1.45 | 1.68 | 1.83 | 1.61 |
| M197C | 1.54 | 0.61 | 0.17 | 0.10 |
| M197D | 1.33 | 0.98 | 1.51 | 1.22 |
| M197G | 0.91 | 1.38 | 1.43 | 1.02 |
| M197H | 1.58 | 1.24 | 1.51 | 1.52 |
| M197L | 1.67 | 1.38 | 1.50 | 1.56 |
| M197T | 1.95 | 1.80 | 1.59 | 1.89 |
| M197V | 1.39 | 1.42 | 1.33 | 1.55 |
| M197I | 1.57 | 1.39 | 1.30 | 1.34 |
| M197S | 1.44 | 1.39 | 1.34 | 1.51 |
| M197N | 1.27 | 1.20 | 1.39 | 1.46 |
| Termamyl^{®} | 1.66 | 1.08 | 0.54 | 0.29 |

### Documentation for improved activity level in the presence of oxidizing agents

All mutants are purified to homogeneity, and the absorption A₂₈₀ is taken as an indication of the protein content. The purified samples were diluted until a value of A₂₈₀ of 0.0014, corresponding to 4 NU/ml in relation to Termamyl^{®}. Under these circumstances the specific activity of each mutant was measured in the presence of a strong oxidizing agent (peracetic acid) and as a comparison in the absence of the strong oxidizing agent. The activities were determined as described in AF 207/1 but with a standard curve constructed at pH 9.0, 60°C instead of at pH 7.3, 37°C. Termamyl^{®} was used as reference in the standard curve. The activity units are therefore referred to as NU*. It clearly appears from Table 2, in which the values of the specific activities are given that the activity level in the environment comprising the strong oxidizing agent in comparison to the control is superior for all mutants, when compared to Termamyl^{®}.

**Table 2**

| | 60°C, pH 9.0 | 60°C, pH 9.0 5 mM peracetic acid |
|---|---|---|
| Termamyl^{®} | 2271 NU* | 528 NU* |
| M197A | 1743 NU* | 1328 NU* |
| M197G | 1708 NU* | 1329 NU* |
| M197T | 1535 NU* | 1256 NU* |
| M197V | 892 NU* | 771 NU* |
| M197Q | 999 NU* | 802 NU* |
| M197F | 514 NU* | 421 NU* |
| M197L | 1515 NU* | 1686 NU* |
| M197H | 792 NU* | 472 NU* |
| M1971 | 1456 NU* | 1276 NU* |
| M197S | 1968 NU* | 1102 NU* |
| M197N | 1750 NU* | 1556 NU* |

### Documentation for improved thermoactivation at moderate low pH values

Termamyl^{®} variants M197A and M197T and Termamyl^{®}, respectively, were purified to homogeneity, and dilution was made according to A₂₈₀ in 50mM Britton-Robinson buffer. Dilution were made to reach A₆₂₀ absorption values between 0.5 and 2 measured in Phasebas assay described in Af 207/1. The respective pH values were adjusted to the indicated pH values between 4 and 10.5. After measurement of the A₆₂₀ values all results were adjusted to A₆₂₀ units/A₂₈₀ (enzyme content), vide Table 3.

The values in the following Table 3 are A₆₂₀ absorptions, which are taken as indications of the activities.

**Table 3**

| A₆₂₀ Phadebas units / A₂₈₀ (enzyme content) | | | | | | | | |
|---|---|---|---|---|---|---|---|---|
| | 37°C | | 60°C | | | | 90°C | |
| pH | Termamyl^{®} | M197A | Termamyl^{®} | M197A | Termamyl^{®} | M197T | Termamyl^{®} | M197A |
| 4.0 | 217 | 113 | 1045 | 464 | 1045 | 177 | 0 | 0 |
| 5.0 | 876 | 903 | 3551 | 5185 | 3551 | 3828 | 9142 | 13272 |
| 6.0 | 1067 | 1088 | 3810 | 5673 | 3810 | 4088 | 8405 | 12566 |
| 7.0 | 1104 | 1080 | 3711 | 5081 | 3711 | 3813 | 6678 | 8936 |
| 8.0 | 1131 | 1061 | 3576 | 4997 | 3576 | 3404 | 3993 | 3965 |
| 9.0 | 1005 | 731 | 2129 | 3256 | 2129 | 1692 | 913 | 191 |
| 10.0 | 685 | 264 | 1937 | 1042 | 1937 | 369 | 0 | 0 |
| 10.5 | 560 | 164 | 1429 | 564 | 1429 | 182 | 0 | 0 |

### Documentation for improved storage stability of the mutants as pseudo prill in the presence of detergent

The purified mutant samples were lyophilized. Detergent and 75°C hot Berol 08 was added under heavy stirring. After hardening of the wax the product is transferred to a freezer and after a couple of hours the products is crushed and mixed with detergent.

The storage was conducted in closed vials at 37°C.

The percentage residual activities after storage under the described conditions are given in the following Table 4.

**Table 4**

| | Days | | | |
|---|---|---|---|---|
| | 0 | 3 | 7 | 14 |
| Termamyl^{®} | 100% | 32% | 16% | 10% |
| M197L | 100% | 56% | 30% | 20% |
| M197T | 100% | 66% | 36% | 23% |
| M197Q | 100% | 56% | 56% | 20% |
| M197F | 100% | 65% | 54% | 34% |

Detergent composition:
9% sodium perborate monohydrate
6% TAED
2% AEO
25% sodium disilicate
2.0% phosphonate
1.2% polycarboxylates
30% trisodium citrate
14.8% sodium carbonate
10% sodium sulfate

### Documentation for improved stability of the mutants in detergent slurries

The samples of purified mutants were incubated with a protein concentration of 1 mg/ml in a suspension of 5% w/w of ADD (ADD is an abbreviation of Automatic Dishwashing Detergent) detergent at 30°C and pH was adjusted to 10.5. ADD contains oxidizing agent (sodium perborate) and TAED.

The reduction in activity of the different mutants was followed during 270 minutes.

The percentage residual activities during incubation are given in the following Table 5 ("0" minutes is an initial measurement after addition of the detergent suspension).

**Table 5**

| | Minutes | | | |
|---|---|---|---|---|
| | 0 | "0" | 60 | 270 |
| Termamyl^{®} | 100% | 39% | 14% | 8% |
| M197L | 100% | 85% | 67% | 61% |
| M197A | 100% | 81% | 44% | 34% |
| M197T | 100% | 90% | 53% | 42% |

## Claims

1. Mutant *Bacillus licheniformis, Bacillus amyloliquefaciens* or *Bacillus stearothermophilus* alpha-amylase **characterised in that** the methionine amino acid residue in position 197 in *Bacillus licheniformis* or *Bacillus amyloliquefaciens* alpha-amylase or in posilion 200 in *Bacillus stearothermophilus* alpha-amylase is exchanged with a Leu, Ile, Asn, Ser, Gln, Asp or Glu.

2. Mutant alpha-amylase according to Claim 1, **characterized in that** the methionine amino acid residue is exchanged with Leu.

3. Detergent **characterized in that** it comprises the mutant alpha-amylase according to Claim 1 or Claim 2.

4. Dish washing agent, **characterized by** the fact that it comprises a mutant alpha-amylase according to Claim I or Claim 2.

## Patentansprüche

1. Mutante alpha-Amylase von *Bacillus licheniformis, Bacillus amyloliquefaciens* oder *Bacillus stearothermophilus,* **dadurch gekennzeichnet, dass** der Aminosäurerest Methionin in Position 197 in der alpha-Amylase von *Bacillus licheniformis* oder *Bacillus amyloliquefaciens* oder in Position 200 in der alpha-Amylase von *Bacillus stearothermophilus* durch ein Leu, Ile, Asn, Ser, Gln, Asp oder Glu ausgetauscht ist.

2. Mutante alpha-Amylase nach Anspruch 1, **dadurch gekennzeichnet, dass** der Aminosäurerest Methionin durch Leu ausgetauscht ist.

3. Detergens, **dadurch gekennzeichnet, dass** es die mutante alpha-Amylase gemäß Anspruch 1 oder Anspruch 2 umfasst.

4. Geschirrspülmittel, **dadurch gekennzeichnet, dass** es eine mutante alpha-Amylase gemäß Anspruch 1 oder Anspruch 2 umfasst.

## Revendications

1. Alpha-amylase mutante de *Bacillus licheniformis, Bacillus amyloliquefaciens,* ou *Bacillus stearothermophilus,* **caractérisée en ce que** le résidu d'acide aminé méthionine en position 197 dans l'alpha-amylase de *Bacillus licheniformis* ou *Bacillus amyloliquefaciens* ou en position 200 dans l'alpha-amylase de *Bacillus stearothermophilus* est échangé avec Leu, Ile, Asn, Ser, Gln, Asp ou Glu.

2. Alpha-amylase mutante selon la revendication 1, **caractérisée en ce que** le résidu d'acide aminé méthionine est échangé avec Leu.

3. Détergent **caractérisé en ce qu'**il comprend l'alpha-amylase mutante selon la revendication 1 ou la revendication 2.

4. Détergent pour vaisselle **caractérisé en ce qu'**il comprend l'alpha-amylase mutante selon la revendication 1 ou la revendication 2.
